# EUROPÄISCHE PATENTSCHRIFT

(11) **EP 0 505 804 B1**
(45) Veröffentlichungstag und Bekanntmachung des Hinweises auf die Patenterteilung: **15.02.1995**
(21) Anmeldenummer: 92103964.0
(22) Anmeldetag: 09.03.1992
(51) Int. Cl.: A61M 25/02

(54) **Fixierpflaster**
Fixing plaster
Pansement de fixation

(30) Priorität: 27.03.1991 DE 9103742 U
(43) Veröffentlichungstag der Anmeldung: 30.09.1992
(73) Patentinhaber: Lohmann GmbH & Co. KG, 56567 Neuwied (DE)
(72) Erfinder: List, Harald, Dr., W-5450 Neuwied 12 (DE)
(74) Vertreter: Flaccus, Rolf-Dieter, Dr.

(56) Entgegenhaltungen:
- FR-A- 830 113
- US-A- 3 927 676
- US-A- 4 074 397
- US-A- 4 460 356
- US-A- 4 534 762

## Beschreibung

Die Neuerung betrifft ein Fixierpflaster, das aus einem mit einer Haftklebeschicht ausgerüsteten Trägermaterial und einer die Haftklebeschicht abdeckenden Schutzschicht besteht sowie mit einer durch vom Rand des Pflasters ausgehende, zur Mitte hin geführte Einschnitte gebildeten hochklappbaren Lasche zum Befestigen medizinischer Hilfsgeräte wie Katheter, Kanülen, Sonden, Drainagen auf der menschlichen Haut.

Die in der medizinischen Therapie gebräuchlichen Hilfsgeräte der aufgeführten Art (im folgenden allgemein als Kanülen bezeichnet) erfordern eine zuverlässige Fixierung. Diese erfolgt in seltenen Fällen durch Annähen, größtenteils jedoch durch Verwendung selbstklebender, teilweise vorgefertigter, gelegentlich auch einzeln hergerichteter Pflasterstreifen. Die bekannten Lösungen sind unbefriedigend.

In der DE 31 05 187 A1 ist eine gattungsgemäße Befestigungsmöglichkeit beschrieben, bei der ein Pflaster mit einem Fixierungsstreifen in Form einer Lasche versehen ist. Diese Lasche entsteht durch zwei gleichgerichtete Einschnitte vom Pflasterrand zur Pflastermitte hin und läßt sich nach dem Hochklappen schraubenlinienförmig um die zu befestigende Kanüle herumwickeln Damit wird zwar die Fixierung der Kanüle erreicht, Stabilität und Sicherheit der Befestigung lassen jedoch zu wünschen übrig.

Aufgabe der Neuerung ist es daher, ein Fixierpflaster der eingangs beschriebenen Art bereitzustellen, das eine einfache, sichere und stabile Fixierung der Kanüle gewährleistet. Diese Aufgabe wird durch ein Fixierpflaster, das aus einem mit einer Haftklebeschicht ausgerüsteten Trägermaterial und einer die Haftklebeschicht abdeckenden Schutzschicht besteht sowie mit einer durch vom Rand des Pflasters ausgehende, zur Mitte hin geführte Einschnitte gebildeten hochklappbaren Lasche zum Befestigen der medizinischen Hilfsgeräte ausgestattet ist, gelöst, das sich dadurch auszeichnet, daß die Lasche durch einen mittigen, bevorzugt über die Länge der Lasche hinausgeführten Einschnitt in zwei Befestigungsstreifen aufgeteilt ist. Die zwei Streifen lassen sich hochklappen und in gegenläufiger Schraubenlinie um die Kanüle herumwickeln. Vorzugsweise wird der mittlere Einschnitt über die Länge der Lasche so weit, beispielsweise um die Breite eines der entstehenden Befestigungs streifen, über die gleich langen seitlichen Einschnitte hinausgeführt, daß die zwei Befestigungsstreifen über zur Erstreckungsrichtung der Lasche schräg und zueinander gegenläufig gerichtete, in Form einer zum Laschenende weisenden Pfeilspitze verlaufende Faltkanten mit seitlicher Komponente spiegelbildlich zueinander auffaltbar sind.

Durch die Unterteilung der durch drei gleichgerichtete, von einer Schmalseite des Fixierpflasters bzw. bei runder Ausführung des Pflasters von einer beliebigen Stelle des Pflasterrandes aus geführte Einschnitte erzeugten Lasche können gegenüber dem bekannten Fixierpflaster zwei Befestigungsstreifen in entgegengesetzter Richtung schraubenlinienförmig um die festzulegende Kanüle gewickelt werden. Durch die schräg zu den Begrenzungskanten der Befestigungsstreifen verlaufenden Faltkanten wird dabei ein im wesentlichen falterfreies Anlegen der Streifen ermöglicht.

Vorteilhaft wird die Breite der aus den zwei Befestigungsstreifen bestehenden Lasche derart festgelegt, daß sie im Bereich der Faltkanten mindestens dem Durchmesser der zu befestigenden Kanüle entspricht. Wird dabei ein leichtes Aufkragen der Innenkanten der Randstreifen des Fixierpflasters im Bereich der Faltkanten in Kauf genommen, so ist auf diese Weise auch die Versorgung stärkerer Kanülen möglich.

Um zu vermeiden, daß durch das Abziehen der aus einem der hierfür bekannten Materialien bestehenden ablösbaren Schutzschicht auch die Klebseite der Befestigungsstreifen freigelegt wird, was zur Erschwerung beim Anlegen der Streifen führen kann, ist bei einer Weiterbildung der Neuerung die Schutzschicht derart unterteilt, daß die Schutzstreifen der beiden Befestigungsstreifen von der übrigen Schutzschicht getrennt und vorzugsweise einzeln abgezogen werden können. Es versteht sich, daß zur Erleichterung der Handhabung die Schutzschicht das eigentliche Pflaster ggf. allseitig überragen kann.

Form und Abmessungen des neuerungsgemäßen Fixierpflasters werden durch den Verwendungszweck vorgegeben. Dabei kann das Trägermaterial aus einem textilen Flächengebilde oder einem geeigneten Folienmaterial bestehen. Als Kleber finden die bekannten hautverträglichen Haftkleber Anwendung.

Anhand der in der Zeichnung dargestellten Ausführungsbeispiele wird die Neuerung näher erläutert. Es zeigt:
- Fig. 1: Draufsicht auf ein neuerungsgemäßes Fixierpflaster vor der Applikation;
- Fig. 2: perspektivische Ansicht eines applizierten Pflasters gemäß Fig. 1;
- Fig. 3: Draufsicht auf ein weiteres neuerungsgemäßes Fixierpflaster vor der Applikation.

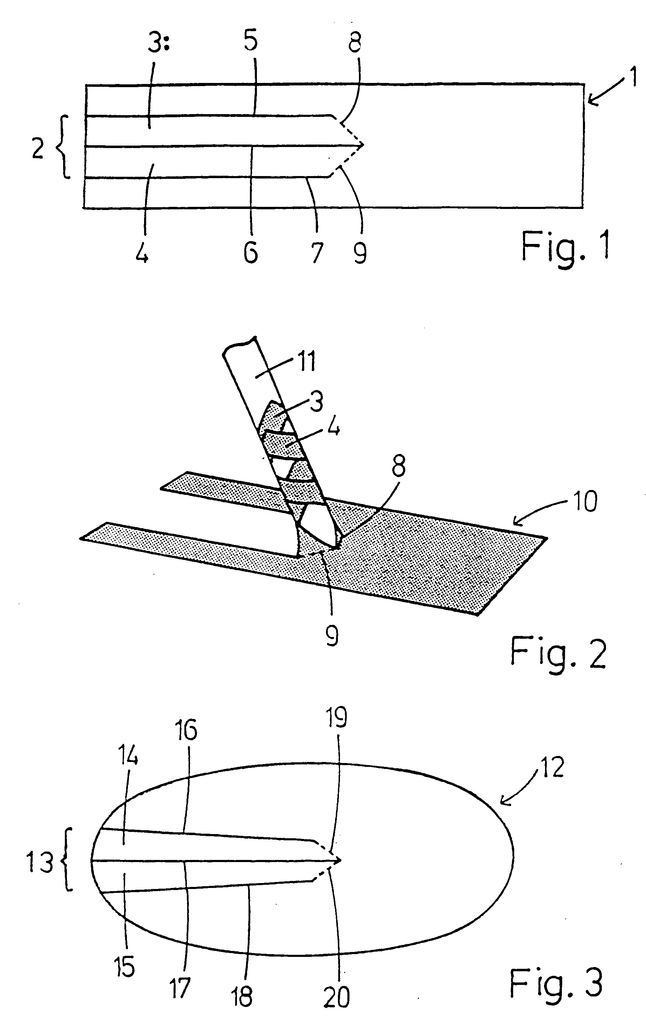

Das in Fig. 1 dargestellte rechteckige Fixierpflaster 1 weist eine aus den Befestigungsstreifen 3 und 4 bestehende Lasche 2 auf, die durch drei gleichgerichtete, von einer der Schmalseiten des Pflasters 1 aus im wesentlichen parallel zu den längslaufenden Seitenkanten des Pflasters zur Pflastermitte hin geführte Einschnitte 5, 6, 7 gebildet wird. Dabei ist der mittlere Einschnitt 6 um einen Betrag über die seitlichen Einschnitte 5, 7 hinausgeführt, der etwa der Breite eines Befestigungsstreifens 3; 4 im Bereich der Faltkanten 8, 9 entspricht. Beim Hochklappen der Befestigungsstreifen 3, 4 bildet sich so jeweils eine zur Einschnittrichtung etwa einen Winkel von ca. 45° bildende Faltkante 8 bzw. 9. Beide Faltkanten 8, 9 verlaufen zueinander so, daß sie zusammen mit dem mittleren Einschnitt 6 etwa die Form eines Pfeiles zeigen. Auf diese Weise ist ein im wesentlichen faltenfreies Anbringen der Befestigungsstreifen 3, 4 an der Kanüle 11 (Fig. 2) möglich.

Darauf hinzuweisen ist hier, daß die zur Bildung der Lasche 2 geführten Einschnitte 5, 7 zwar im allgemeinen im wesentlichen parallel zu den Längskanten des Fixierpflasters verlaufen, daß sie aber auch zu diesen einen dann vorzugsweise zum mittleren Einschnitt spiegelsymmetrischen spitzen Winkel bilden können. Die spitzen Winkel, welche die beiden äußeren Einschnitte 5, 7 zum mittleren Einschnitt 6 bilden, können so liegen, daß die Breite der Befestigungsstreifen am Beginn der Einschnitte 5-7 größer ist als im Bereich der Faltkanten oder umgekehrt. In jedem Falle ist die Auswahl vom Länge, Breite und Breitenverlauf der Befestigungsstreifen von dem konkreten Verwendungszweck des neuerungsgemäßen Fixierpflasters 1 abhängig.

Fig. 2 zeigt in perspektivischer Sicht ein appliziertes Fixierpflaster 10 mit einer Kanüle 11, die vermittels der in einander entgegengesetzter Richtung um sie gewickelten Befestigungsstreifen 3, 4 festgelegt ist. Dabei ist das Pflaster 10 bis zu dem Faltkanten 8, 9 soweit an die Kanüle 11 herangeschoben, daß letztere im dem durch die Faltkanten 8, 9 der hochgeklappten Befestigungsstreifen 3, 4 gebildeten Winkel zu liegen kommt. Damit ist es möglich, die schräg zur Seite abklappbaren Befestigungsstreifen 3, 4 insbesondere dann faltenfrei an die Kanüle 11 anzulegen, wenn diese zu der Klebeebene des Pflasters 10 ebenfalls einen Winkel - vorzugsweise im Bereich von ca. 45° - bildet. Bei dieser Art der Befestigung erhält die Kanüle 11 einen sicheren und stabilen Halt.

In Fig. 3 ist ein Fixierpflaster 1 vor der Applikation gezeigt, das ovale Form hat. Die im wesentlichen in Richtung der langen Achse des Ovals verlaufenden, zur Bildung der Befestigungsstreifen 14, 15 geführten Einschnitte 16-18 laufen nicht parallel zueinander, sondern so, daß sich der Abstand der die Lasche 13 bildenden äußeren Einschnitte 16, 18 vom Beginn der Einschnitte 16, 18 bis zu ihrem Ende stetig verringert. In gleicher Weise können die äußeren Einschnitte 16, 18 parallel zueinander und zum mittleren Einschnitt 17 oder auseinanderlaufen. Vorzugsweise ist jedoch auch hier der mittlere Einschnitt 17 über die beiden äußeren Einschnitte 16, 18 hinausgeführt, so daß zum mittleren Einschnitt - vorzugsweise in der vorhergehend beschriebenen Weise verlaufende - schräge Faltkanten entstehen.

Es versteht sich, daß bei Beibehaltung der in zwei unabhängige Befestigungsstreifen 3, 4 bzw. 14, 15 aufgeteilten Lasche 2 bzw. 13 Form und Größe des neuerungsgemäßen Fixierpflasters 1 bzw. 12 ebenso wie Form und Abmessungen der Lasche 2 bzw. 13 in beliebiger Weise an die jeweiligen Bedürfnisse angepaßt werden können.

Es hat sich gezeigt, daß das neuerungsgemäße Fixierpflaster in der Handhabung besonders einfach und sicher ist und auch einfach und preiswert hergestellt werden kann. Mit ihm ist eine sichere und stabile Befestigungsmöglichkeit für medizinische Hilfsgeräte der eingangs aufgeführten Art bereitsgestellt.

### ZUSAMMENSTELLUNG DER BEZUGSZEICHEN

- 1: Fixierpflaster
- 2: Lasche
- 3: Befestigungsstreifen
- 4: Befestigungsstreifen
- 5: Einschnitt
- 6: Einschnitt
- 7: Einschnitt
- 8: Faltkante
- 9: Faltkante
- 10: Fixierpflaster
- 11: Kanüle, Hilfsgerät
- 12: Fixierpflaster
- 13: Lasche
- 14: Befestigungsstreifen
- 15: Befestigungsstreifen
- 16: Einschnitt
- 17: Einschnitt
- 18: Einschnitt
- 19: Faltkante
- 20: Faltkante

## Patentansprüche

1. Fixierpflaster (1,10,12) aus einem mit einer Haftklebeschicht ausgerüsteten Trägermaterial und einer die Haftklebeschicht abdeckenden Schutzschicht mit einer durch vom Rand des Pflaster (1,10,12) ausgehende, zur Mitte hin geführte Einschnitte (5,6;16,18) gebildeten hochklappbaren Lasche (2,13) zum Befestigen medizinischer Hilfsgeräte wie Kanülen auf der menschlichen Haut,
**dadurch gekennzeichnet, daß**
die Lasche (2,13) durch einen mittigen, bevorzugt über die Lange der Lasche (2,13) hinausgeführten Einschnitt (6,17) in zwei Befestigungsstreifen (3,4;14,15) aufgeteilt ist.

2. Fixierpflaster nach Anspruch 1,
**dadurch gekennzeichnet, daß**
der mittige Einschnitt (6; 17) so weit, vorzugsweise etwa um die Breite eines Befestigungsstreifens (3, 4; 14, 15) im Endbereich der Einschnitte (5-7; 16-18), über die gleich langen seitlichen Einschnitte (5, 7; 16, 18) hinausgeführt ist, daß die zwei Befestigungsstreifen (3, 4; 14, 15) über zu ihrer Erstreckungsrichtung schräg und zueinander gegenläufig verlaufende Faltkanten (8, 9; 19, 20) mit seitlicher Komponente auffaltbar sind.

3. Fixierpflaster nach Anspruch 1 oder 2,
**dadurch gekennzeichnet, daß**
die Befestigungsstreifen (3, 4; 14, 15) durch drei gleichgerichtete, vom Rand einer Schmalseite des Fixierpflasters (1; 12) bzw. bei rundem Fixierpflaster vom einer beliebigen Stelle des Pflasterrandes ausgehende und im wesentlichen bis zur Pflastermitte geführte Einschnitte (5-7; 16-18) gebildet sind.

4. Fixierpflaster nach einem der Ansprüche 1 bis 3,
**dadurch gekennzeichnet, daß**
die Breite der durch die beiden Befestigungsstreifen (3, 4; 14, 15) gebildeten Lasche (2; 13) im Bereich der Faltkanten (8, 9; 19, 20) mindestens dem Durchmesser des festzulegenden medizinischen Hilfsgerätes (11) entspricht.

5. Fixierpflaster nach einem der Ansprüche 1 bis 4,
**dadurch gekennzeichnet, daß**
die zur Bildung der Lasche (2) geführten Einschnitte (5, 7; 16, 18) im wesentlichen parallel zu den Längskanten des Fixierpflasters (1; 12) verlaufen.

6. Fixierpflaster nach einem der Ansprüche 1 bis 4,
**dadurch gekennzeichnet, daß**
die zur Bildung der Lasche (2) geführten Einschnitte (5, 7; 16, 18) zu dem mittleren Einschnitt (6; 17) einen zum mittleren Einschnitt (6, 17) spiegelsymmetrischen spitzen Winkel bilden.

7. Fixierpflaster nach Anspruch 6,
**dadurch gekennzeichnet, daß**
die von den beiden äußeren Einschnitten (5, 7; 16, 18) zum mittleren Einschnitt (6; 17) gebildeten spitzen Winkel so liegen, daß die Breite der Befestigungsstreifen (3, 4; 14, 15) am Beginn der Einschnitte (5-7; 16-18) größer ist als im Bereich der Faltkanten (8, 9; 19, 20) oder umgekehrt.

8. Fixierpflaster nach einem der Ansprüche 1 bis 7,
**dadurch gekennzeichnet, daß**
das Trägermaterial des Fixierpflasters (1; 12) ein textiles Flächengebilde oder ein geeignetes Folienmaterial ist.

9. Fixierpflaster nach einem der Ansprüche 1 bis 8,
**dadurch gekennzeichnet, daß**
die Schutzschichten des Fixierteils (10) des Fixierpflasters (1; 12) und der Befestigungsstreifen (3, 4; 14, 15) unabhängig voneinander abziehbar sind.

## Claims

1. A fixation plaster (1, 10, 12) comprising a carrier material provided with a pressure-sensitive adhesive layer and a protective layer covering said pressure-sensitive adhesive layer, and having a lift-up type (2, 13) flap formed by cuts (5, 6; 16, 18) extending from the edge of the plaster (1, 10, 12) towards the center, for fastening medical accessories, such as cannulae, to the human skin, characterized in that said flap (2; 13) is divided into two fastening strips (3, 4; 14, 15) by a centric cut (6; 17) which preferably extends beyond the length of the flap (2; 13).

2. A fixation plaster according to claim 1, characterized in that the centric cut (6; 17) extends beyond the lateral cuts (5, 7; 16, 18) of equal length by such a distance, preferably by approximately the width of a fastening strip (3, 4; 14, 15) in the end portion of the cuts (5-7; 16-18), that the two fastening strips (3, 4; 14, 15) can be folded with lateral component at the folding edges (8, 9; 19, 20) running diagonally to the direction of said fastening strips and in opposite direction to each other.

3. A fixation plaster according to claims 1 or 2, characterized in that the fastening strips (3, 4; 14, 15) are formed by three unidirectional cuts (5-7; 16-18) starting from the edge of a narrow side of the fixation plaster (1; 12) or, in case of a round fixation plaster, from any point of the plaster edge and running substantially up to the center of the plaster.

4. A fixation plaster according to any one of claims 1 to 3, characterized in that the width of the flap (2; 13), which is formed by the two fastening strips (3, 4; 14, 15), in the region of the folding edges (8, 9; 19, 20) corresponds at least to the diameter of the medical accessories (11) to be fixed.

5. A fixation plaster according to any one of claims 1 to 4, characterized in that the cuts (5, 7; 16, 18) forming the flap (2) run substantially parallel to the longitudinal edges of the fixation plaster (1; 12).

6. A fixation plaster according to any one of claims 1 to 4, characterized in that the cuts (5, 7; 16, 18) forming the flap (2) form a sharp angle to the central cut (6; 17) which, with respect to said central cut (6; 17), is a symmetric sharp angle.

7. A fixation plaster according to claim 6, characterized in that the sharp angles which are formed by the two outer cuts (5, 7; 16, 18) towards the central cut (6, 17) are placed such that the width of the fastening strips (3, 4; 14, 15) at the beginning of the cuts (5-7; 16-18) is larger than in the region of the folding edges (8, 9; 19, 20), or vice versa.

8. A fixation plaster according to any one of claims 1 to 7, characterized in that the carrier material of the fixation plaster (1; 12) is a textile fabric or a suitable film or sheet material.

9. A fixation plaster according to any one of claims 1 to 8, characterized in that the protective layers of the fixing portion (10) of the fixation plaster (1; 12) and of the fastenings strips (3, 4; 14, 15) may be removed independently of each other.

## Revendications

1. Emplâtre de fixation (1, 10, 12) qui est constitué d'un matériau de support doté d'une couche de colle d'adhérence et d'une couche de protection recouvrant la couche de colle d'adhérence, et comporte une languette (2,13) pour la fixation sur la peau humaine d'accessoires médicaux tels que cathéters, canules, sondes, drains, qui peut être rabattue vers le haut et est formée par des découpes (5, 6; 16, 18) partant du bord de l'emplâtre (1, 10, 12) et s'étendant jusqu'au milieu, caractérisé en ce que la languette (2, 13) est divisée en deux bandes de fixation (3, 4; 14, 15) par une découpe médiane (6, 17) s'étendant de préférence sur toute la longueur de la languette (2, 13).

2. Emplâtre de fixation selon la revendication 1, caractérisé en ce que, dans la région d'extrémité des découpes (5, 7; 16, 18), la découpe médiane (6; 17) se prolonge suffisamment loin au-delà des découpes latérales (5, 7; 16, 18) de même longueur, de préférence sensiblement de la largeur d'une bande de fixation (3, 4; 14, 15), pour que les deux bandes de fixation (3, 4; 14, 15) puissent être repliées sur des arêtes de pliage (8, 9; 19, 20) comportant une composante latérale et s'étendant obliquement l'une vers l'autre par rapport à la direction de leur extension.

3. Emplâtre de fixation selon la revendication 1 ou 2, caractérisé en ce que les bandes de fixation (3, 4; 14, 15) sont formées par trois découpes (5, 7; 16, 18) orientées dans le même sens, partant du bord d'un côté étroit de l'emplâtre de fixation (1; 12) ou d'un emplacement quelconque du bord de l'emplâtre dans le cas d'un emplâtre de fixation rond, et s'avançant essentiellement jusqu'au milieu de l'emplâtre.

4. Emplâtre de fixation selon l'une des revendications 1 à 3, caractérisé en ce que, dans la région des arêtes de pliage (8, 9; 19, 20), la largeur de la languette (2; 13) formée par les deux bandes de fixation (3, 4; 14, 15) correspond au moins au diamètre de l'accessoire médical (11) à fixer.

5. Emplâtre de fixation selon l'une des revendications 1 à 4, caractérisé en ce que les découpes (5, 7; 16, 18) réalisées pour former la languette (2) s'étendent essentiellement parallèlement aux bords longitudinaux de l'emplâtre de fixation (1; 12).

6. Emplâtre de fixation selon l'une des revendications 1 à 4, caractérisé en ce que les découpes (5, 7; 16, 18) réalisées pour former la languette (2) forment avec la découpe médiane (6; 17) un angle aigu à symétrie spéculaire par rapport à la découpe médiane (6; 17).

7. Emplâtre de fixation selon la revendication 6, caractérisé en ce que les angles aigus formés par les deux découpes extérieures (5, 7; 16, 18) avec la découpe médiane (6; 17) sont situés de telle sorte que la largeur des bandes de fixation (3, 4; 14, 15) est plus grande au début des découpes (5, 7; 16, 18) que dans la région des arêtes de pliage (8, 9; 19, 20) ou inversement.

8. Emplâtre de fixation selon l'une des revendications 1 à 7, caractérisé en ce que le matériau de support de l'emplâtre de fixation (1; 12) est un produit plat tissé ou un matériau en feuille approprié.

9. Emplâtre de fixation selon l'une des revendications 1 à 8, caractérisé en ce que les couches de protection de la partie de fixation (10) de l'emplâtre de fixation (1; 12) et des bandes de fixation (3, 4; 14, 15) peuvent être arrachées indépendamment l'une de l'autre.
